# EUROPEAN PATENT APPLICATION

(11) **EP 4 424 679 A1**
(43) Date of publication of application: **04.09.2024**
(21) Application number: 22885924.5
(22) Date of filing: 25.10.2022
(51) Int. Cl.: C07D 307/60

(54) **METHOD FOR PREPARING SUCCINIC ANHYDRIDE BY MEANS OF HYDROGENATION OF MALEIC ANHYDRIDE AND PRODUCTION SYSTEM**

(30) Priority: 28.10.2021 CN 202111260918
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: QIAO, Kai, Dalian, Liaoning 116045 (CN); YANG, Xiuna, Dalian, Liaoning 116045 (CN); ZHOU, Feng, Dalian, Liaoning 116045 (CN); LI, Lanpeng, Dalian, Liaoning 116045 (CN); RUAN, Zonglin, Dalian, Liaoning 116045 (CN)
(74) Representative: Hoffmann Eitle
(86) International application number: PCT/CN2022/127300
(87) International publication number: WO 2023/072047

(57) **Abstract**

Disclosed is a process for preparing succinic anhydride by hydrogenation of maleic anhydride comprising steps of: 1) mixing a maleic anhydride solution with hydrogen to obtain a first liquid phase feed with hydrogen dispersed in the liquid phase; 2) carrying out a first hydrogenation reaction by passing the first liquid phase feed from bottom to top through fixed bed layer(s) of a first maleic anhydride hydrogenation catalyst arranged in a first reaction unit under first hydrogenation reaction conditions to obtain a first reaction effluent containing succinic anhydride; 3) mixing the first reaction effluent from the first reaction unit with make-up hydrogen to obtain a second liquid phase feed with hydrogen dispersed in the liquid phase; and 4) carrying out a second hydrogenation reaction by passing the second liquid phase feed from bottom to top through fixed bed layer(s) of a second maleic anhydride hydrogenation catalyst arranged in a second reaction unit under second hydrogenation reaction conditions to obtain a second reaction effluent containing succinic anhydride. The process can effectively solve the problems of concentrated heat release and easy generation of local hot spots during the hydrogenation of maleic anhydride, and maintain higher conversion rate and selectivity in the hydrogenation of maleic anhydride.

## Description

### Technical Field

The application relates to the technical field of succinic anhydride production, and particularly relates to a process and a production system for preparing succinic anhydride by hydrogenation of maleic anhydride.

### Background Art

At present, production methods of succinic anhydride mainly comprise a succinic acid dehydration method, a biological fermentation method and a maleic anhydride catalytic hydrogenation method. Among them, the maleic anhydride (also known as maleic acid anhydride) catalytic hydrogenation method is the method for producing succinic anhydride with the highest conversion rate and the highest product yield, making it the most suitable method for large-scale industrialization. However, the hydrogenation of maleic anhydride to produce succinic anhydride is a strongly exothermic reaction (ΔH = 128 kJ/mol). The reaction heat cannot be timely removed by using the conventional trickle bed hydrogenation, resulting in uncontrollable temperatures in the reaction process, local hot spots in the catalyst bed layer(s), serious side reactions and the like. This makes it impossible to control the safety, conversion rate, and selectivity of the reaction process.

CN103570650A provides a process for continuously producing succinic anhydride and coproducing succinic acid by hydrogenation of maleic anhydride. This method employs a two-stage hydrogenation reactor, wherein the first-stage hydrogenation reactor is a fixed bed reactor with hydrogen and reaction liquid entering from the bottom and exiting from the top, and the second-stage hydrogenation reactor is a trickle bed reactor with hydrogen and reaction liquid entering from the top and exiting from the bottom. External circulation is used to remove the reaction heat, aiming to control the average operating temperature of the entire reactor, and to ensure a balanced temperature within the reactor. In this process, a flow mode with hydrogen and reaction liquid flowing cocurrently and upward is used in the first-stage reactor. Due to the particularity of the large amount of heat released during the hydrogenation reaction of maleic anhydride, conventional techniques cannot ensure uniform mixing and distribution of materials, nor can they ensure uniform reaction and address the problem of local hot spots. In the second-stage reactor, a flow mode of the trickle bed reactor with cocurrent and downward flow is even less able to ensure timely removing reaction heat and addressing the problem of local hot spots.

CN105801536B provides a method for preparing succinic anhydride by liquid phase selective hydrogenation of maleic anhydride, wherein succinic anhydride is prepared by the liquid phase hydrogenation reaction using a two-stage low-temperature and low-pressure reaction process with two reactors: a first-stage reactor and a second-stage reactor, respectively, which are used in series. Maleic anhydride, solvent and hydrogen are fed into the first-stage reactor to carry out partial catalytic selective hydrogenation. After the reaction, the residual maleic anhydride, the mixed solution material of generated succinic anhydride and solvent are fed into the second-stage reactor to carry out complete catalytic selective hydrogenation. The product from the second-stage reactor is then subjected to gas-liquid separation and rectification to obtain the succinic anhydride product. In this method, liquid phase hydrogenation method of hydrogen and reaction liquid is used in the two-stage reactors. Due to the particularity of the large amount of heat released during the hydrogenation reaction of maleic anhydride, on one hand, it is difficult to address problems of both the concentrated heat release and local hot spots during the early stages of the reaction; on the other hand, it is also difficult to ensure avoiding problems of deep hydrogenation and reduced selectivity caused by back mixing of the materials and uneven distribution during the later stages of the reaction.

### Disclosure of the Invention

Aiming at the defects of the prior art, the present application provides a process and a production system for preparing succinic anhydride by hydrogenation of maleic anhydride, which can effectively solve the problems of concentrated heat release and easy generation of local hot spots during the hydrogenation of maleic anhydride and keep relatively high conversion rate and selectivity of maleic anhydride hydrogenation.

In order to achieve the above object, the present application provides in one aspect a process for preparing succinic anhydride by hydrogenation of maleic anhydride, comprising steps of:
1) mixing a maleic anhydride solution with hydrogen to obtain a first liquid phase feed with hydrogen dispersed in the liquid phase, wherein the ratio of the volume flow rate of the hydrogen in Nm³/h to the volume flow rate of the maleic anhydride solution in m³/h is in a range of 5: 1 to 50: 1;
2) carrying out a first hydrogenation reaction by passing the first liquid phase feed from bottom to top through fixed bed layer(s) of a first maleic anhydride hydrogenation catalyst arranged in a first reaction unit under first hydrogenation reaction conditions to obtain a first reaction effluent containing succinic anhydride, wherein the first hydrogenation reaction conditions include: a reaction temperature of 40-200 °C; a reaction pressure of 0.5-10.0 MPa; and a liquid hourly space velocity of 5.0-20.0 h⁻¹;
3) mixing the first reaction effluent from the first reaction unit with make-up hydrogen to obtain a second liquid phase feed with hydrogen dispersed in the liquid phase, wherein the ratio of the volume flow rate of the make-up hydrogen in Nm³/h to the volume flow rate of the maleic anhydride solution used in step 1) in m³/h is in a range of 1: 1 to 20: 1; and
4) carrying out a second hydrogenation reaction by passing the second liquid phase feed from bottom to top through fixed bed layer(s) of a second maleic anhydride hydrogenation catalyst arranged in a second reaction unit under second hydrogenation reaction conditions to obtain a second reaction effluent containing succinic anhydride, wherein the second hydrogenation reaction conditions include: a reaction temperature of 40-150 °C; a reaction pressure of 0.5-10.0 MPa; and a liquid hourly space velocity of 0.1-4.0 h⁻¹.

Preferably, the first reaction unit comprises one or more up-flow fixed bed reactor(s) having arranged therein one or more fixed bed layer(s) of the first maleic anhydride hydrogenation catalyst, wherein the height-to-diameter ratio of each up-flow fixed bed reactor is independently in a range of 3-20; and the second reaction unit comprises one or more up-flow fixed bed reactor(s) having arranged therein one or more fixed bed layer(s) of the second maleic anhydride hydrogenation catalyst, wherein the height-to-diameter ratio of each up-flow fixed bed reactor in the second reaction unit is less than that of the up-flow fixed bed reactor in the first reaction unit and is in a range of 0.1-2.5.

In another aspect, the present application provides a production system for preparing succinic anhydride by hydrogenation of maleic anhydride, comprising a first gas-liquid mixer, a first reaction unit, a second gas-liquid mixer, a second reaction unit and a fractionation device which are connected in sequence, wherein the first and second gas-liquid mixers are each provided with an inlet for liquid, an inlet for gas and an outlet for liquid phase mixture, the first and second reaction units each independently comprise one or more series-connected and/or parallel-connected up-flow fixed bed reactor(s) having arranged therein one or more fixed bed layer(s) of a maleic anhydride hydrogenation catalyst, each up-flow fixed bed reactor is provided with an inlet for hydrogenation feed and an outlet for hydrogenation product, and the fractionation device is provided with an inlet and an outlet for succinic anhydride product,
wherein the inlet for liquid of the first gas-liquid mixer is communicated with a maleic anhydride solution source, the inlet for gas is communicated with a hydrogen source, the outlet for liquid phase mixture is communicated with the inlet for hydrogenation feed of the up-flow fixed bed reactor(s) in the first reaction unit, the outlet for hydrogenation product of the up-flow fixed bed reactor(s) in the first reaction unit is communicated with the inlet for liquid of the second gas-liquid mixer, the inlet for gas of the second gas-liquid mixer is communicated with the hydrogen source, the outlet for liquid phase mixture is communicated with the inlet for hydrogenation feed of the up-flow fixed bed reactor(s) in the second reaction unit, the outlet for hydrogenation product of the up-flow fixed bed reactor(s) in the second reaction unit is communicated with the inlet of the fractionating device,
and the height-to-diameter ratio of each up-flow fixed bed reactor in the first reaction unit is independently in a range of 3-15, and the height-to-diameter ratio of each up-flow fixed bed reactor in the second reaction unit is less than that of the up-flow fixed bed reactor in the first reaction unit and is in a range of 0.1-2.5.

The process and the production system for preparing succinic anhydride by hydrogenation of maleic anhydride have the following characteristics:
1) a two-stage up-flow liquid phase hydrogenation reaction process is used to divide the maleic anhydride hydrogenation reaction process into two stages, and different process parameters are used according to the characteristics of the reaction processes in different stages, thereby arriving at the goals of effectively controlling the overall reaction heat and achieving the optimal overall reaction effect;
2) the hydrogenation reaction of the first stage is a rapid hydrogenation reaction carried out under high space velocity conditions. In the reaction of the first stage, the initial concentration of maleic anhydride is high, and the reaction rate is fast, making it easier to generate local hot spots. The first stage is carried out using the up-flow reaction mode under high space velocity conditions, which not only reduces the residence time of maleic anhydride, maximizes the reduction of side reactions and local hot spots, but also effectively prevents catalyst coking by keeping the catalyst in a slightly expanded state;
   in a preferred embodiment, the hydrogenation reaction of the first stage is carried out in an up-flow fixed bed reactor with a large height-to-diameter ratio, which can increase the flow rate of streams in the reactor, intensify turbulence of streams and heat transfer by diffusion, and also enable the streams in the reactor to be in a nearly plug flow state, reducing axial backmixing and thereby reducing side reactions;
   the rapid hydrogenation reaction mode in the first stage ensures efficient mass transfer while achieving a suitable degree of reaction conversion rate, which is more favorable for heat transfer by diffusion, and thereby effectively solves the problems of concentrated heat release and generation of local hot spots in the early stage of reaction;
3) the hydrogenation reaction of the second stage is a mild liquid phase hydrogenation reaction carried out under low space velocity conditions. In the reaction of the second stage, the concentration of maleic anhydride is low, and the reaction rate is low. Particularly, when there is a small amount of residual maleic anhydride, it is difficult to achieve complete conversion. A relatively long residence time is required to achieve a higher conversion rate. The second stage is carried out using the up-flow reaction mode, which not only has high mass transfer reaction efficiency, but also allows the catalyst to be in a slightly expanded state, preventing problems of local hot spots and catalyst coking caused by long residence time;
   in a preferred embodiment, the hydrogenation reaction of the second stage is carried out in an up-flow fixed bed reactor with a less height-to-diameter ratio than that of the up-flow fixed bed reactor used in the first stage, resulting in a relatively low flow rate of streams in the reactor of the second stage, with no significant turbulence of the streams, a more stable catalyst bed, and less prone of the streams to problems such as short-circuit flow, bubbly flow, and the like, effectively ensuring reaction efficiency and conversion capacity;
   the mild hydrogenation reaction process of the second stage is cooperated with the rapid hydrogenation reaction process of the first stage to ensure ideal conversion rates while effectively controlling the occurrence of side reactions; and
4) the rapid hydrogenation reaction process of the first stage and the mild hydrogenation reaction process of the second stage are both stable whole-liquid-phase hydrogenation reaction processes, wherein the liquid phase in the reactor is a continuous phase and the gas phase is a dispersed phase, and the reactor is in a stable whole-liquid-phase hydrogenation reaction state from the inlet(s) to the outlet(s), which contributes to reduce the pulsation of the catalyst bed layer(s), prevent severe wear of catalyst particles, and ensure higher mass transfer efficiency.

Additional features and advantages of the present application will be set forth in the detailed description which follows.

### Brief Description of the Drawings

The drawings, constituting a part of the present description, are provided to help the further understanding of the present application, and should not be considered to be limiting. The present application can be interpreted with reference to the drawings in combination with the detailed description hereinbelow. In the drawings:

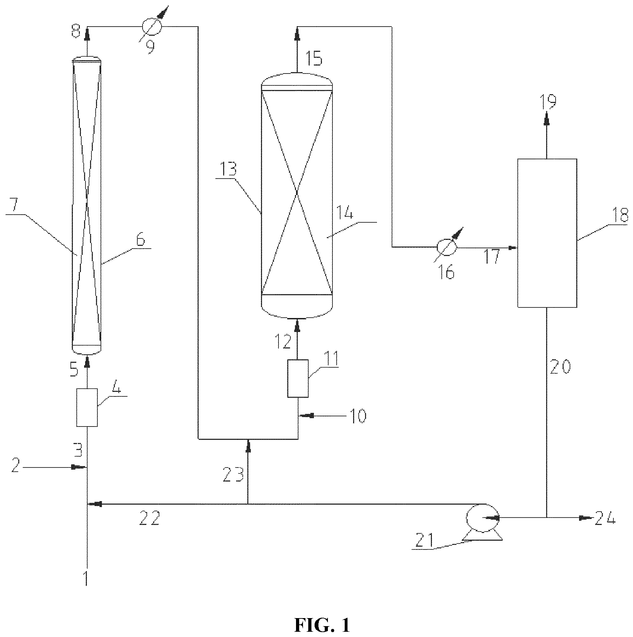

FIG. 1 is a schematic diagram of a preferred embodiment of a production system for preparing succinic anhydride by hydrogenation of maleic anhydride according to the present application.

### Detailed Description of the Invention

The present application will be further described hereinafter in detail with reference to specific embodiments thereof in combination with the accompanying drawings. It should be noted that the specific embodiments described herein are provided for illustration and explanation purposes only, and are not intended to be limiting in any manner.

Any specific numerical value, including the endpoints of a numerical range, described in the context of the present application is not restricted to the exact value thereof, but should be interpreted to further encompass all values close to said exact value, for example all values within ±5% of said exact value. Moreover, regarding any numerical range described herein, arbitrary combinations can be made between the endpoints of the range, between each endpoint and any specific value within the range, or between any two specific values within the range, to provide one or more new numerical range(s), where these new numerical range(s) should also be deemed to have been specifically described in the present application.

Unless otherwise stated, the terms used herein have the same meaning as commonly understood by those skilled in the art; and if the terms are defined herein and their definitions are different from the ordinary understanding in the art, the definition provided herein shall prevail.

In the context of the present application, in addition to those matters explicitly stated, any matter or matters not mentioned are considered to be the same as those known in the art without any change. Moreover, any of the embodiments described herein can be freely combined with another one or more embodiment(s) described herein, and the technical solutions or ideas thus obtained are considered as part of the original disclosure or original description of the present application, and should not be considered to be a new matter that has not been disclosed or anticipated herein, unless it is clear to the person skilled in the art that such a combination is obviously unreasonable.

All of the patent and non-patent documents cited herein, including but not limited to textbooks and journal articles, are hereby incorporated by reference in their entirety.

As described above, in a first aspect, the present application provides a process for preparing succinic anhydride by hydrogenation of maleic anhydride, comprising steps of:
1) mixing a maleic anhydride solution with hydrogen to obtain a first liquid phase feed with hydrogen dispersed in the liquid phase, wherein the ratio of the volume flow rate of the hydrogen in Nm³/h to the volume flow rate of the maleic anhydride solution in m³/h is in a range of 5: 1 to 50: 1, preferably 10: 1 to 30: 1;
2) carrying out a first hydrogenation reaction by passing the first liquid phase feed from bottom to top through fixed bed layer(s) of a first maleic anhydride hydrogenation catalyst arranged in a first reaction unit under first hydrogenation reaction conditions to obtain a first reaction effluent containing succinic anhydride, wherein the first hydrogenation reaction conditions include: a reaction temperature of 40-200 °C, preferably 50-150 °C; a reaction pressure of 0.5-10.0 MPa, preferably 1-5.0 MPa; and a liquid hourly space velocity of 5.0-20.0 h⁻¹, preferably 6.0-15.0 h⁻¹;
3) mixing the first reaction effluent from the first reaction unit with make-up hydrogen to obtain a second liquid phase feed with hydrogen dispersed in the liquid phase, wherein the ratio of the volume flow rate of the make-up hydrogen in Nm³/h to the volume flow rate of the maleic anhydride solution used in step 1) in m³/h is in a range of 1: 1 to 20: 1, preferably 5: 1 to 15: 1; and
4) carrying out a second hydrogenation reaction by passing the second liquid phase feed from bottom to top through fixed bed layer(s) of a second maleic anhydride hydrogenation catalyst arranged in a second reaction unit under second hydrogenation reaction conditions to obtain a second reaction effluent containing succinic anhydride, wherein the second hydrogenation reaction conditions include: a reaction temperature of 40-150 °C, preferably 50-80 °C; a reaction pressure of 0.5-10.0 MPa, preferably 1-5.0 MPa; and a liquid hourly space velocity of 0.1-4.0 h⁻¹, preferably 0.5-2.5 h⁻¹.

In the process of the present application, the first liquid phase feed and the second liquid phase feed are both streams obtained by uniformly mixing and dispersing hydrogen in a liquid phase (maleic anhydride solution and/or maleic anhydride hydrogenation product), wherein the liquid phase is a continuous phase, the hydrogen is a dispersed phase, and the physical state of the streams is similar to the liquid phase.

In the process of the present application, the first hydrogenation reaction in step 2) and the second hydrogenation reaction in step 4) are both stable whole-liquid-phase hydrogenation reaction processes, wherein the so-called "whole-liquid-phase hydrogenation" means that, compared with the trickle bed hydrogenation reaction process, the liquid phase in the reactor is a continuous phase and the gas phase is a dispersed phase, while the gas phase in the trickle bed is a continuous phase and the liquid phase is a dispersed phase. In the present application, the formation of a stable whole-liquid-phase hydrogenation reaction process from the inlet(s) to the outlet(s) of the reactor allows most or all of hydrogen to dissolve and disperse in the liquid phase, which contributes to ensure higher mass transfer reaction efficiency, reduce pulsations of the catalyst bed layer(s), and prevent severe wear of catalyst particles.

In the process of the present application, the formation of the first liquid phase feed and the second liquid phase feed, i.e. the uniform dispersion of hydrogen into the liquid phase, can be achieved by mixing with a mixer, which may be selected from a static mixer, an ejector mixer, a mechanical shear mixer, an impingement mixer, a microchannel mixer, or combinations thereof.

In a preferred embodiment, the maleic anhydride solution used in step 1) has a maleic anhydride content of 0.03-0.3 g/mL, preferably 0.05-0.2 g/mL. Further preferably, the solvent used in the maleic anhydride solution is selected from benzene, toluene, xylene, acetone, tetrahydrofuran, γ-butyrolactone, methyl acetone, cyclohexanone, ethyl acetate, diethyl succinate, ethylene glycol monomethyl ether, or combinations thereof.

In the process of the present application, the hydrogen used may be hydrogen having a purity of more than 90 (v)%, preferably 99.9% pure hydrogen.

In a preferred embodiment, the first reaction unit comprises one or more (e.g. 1-5) up-flow fixed bed reactor(s) having arranged therein one or more (e.g. 1-5) fixed bed layer(s) of the first maleic anhydride hydrogenation catalyst, wherein the height-to-diameter ratio of each up-flow fixed bed reactor is independently in a range of 3-15, preferably in a range of 4-10. For example, the first reaction unit comprises one reactor, and the reactor is provided with 1-4 catalyst bed layers.

In this preferred embodiment, the first hydrogenation reaction in step 1) is a rapid hydrogenation reaction carried out under conditions of a relatively high space velocity and a larger reactor height-to-diameter ratio. Due to the high initial concentration of maleic anhydride and fast reaction rate in the early stage of the reaction, it is easier to generate local hot spots. The up-flow reaction mode operated under high space velocity conditions can not only reduce the residence time of maleic anhydride, maximizing the reduction of side reactions and local hot spots, but also allow the catalyst to be in a slightly expanded state, effectively preventing catalyst coking. Meanwhile, the reactor with a large height-to-diameter ratio can increase the flow rate of streams in the reactor, intensify the turbulence of streams and heat transfer by diffusion, and also enable the streams in the reactor to be in a nearly plug flow state, reducing axial backmixing and thereby reducing side reactions. The rapid hydrogenation reaction mode in step 1) ensures efficient mass transfer while achieving a suitable degree of reaction conversion rate, which is more favorable for heat transfer by diffusion, and effectively solves the problems of concentrated heat release and generation of local hot spots in the early stage of the reaction.

In a preferred embodiment, the second reaction unit comprises one or more (e.g. 1-5) up-flow fixed bed reactor(s) having arranged therein one or more (e.g. 1-5) fixed bed layer(s) of the second maleic anhydride hydrogenation catalyst, wherein the height-to-diameter ratio of each up-flow fixed bed reactor in the second reaction unit is less than that of the up-flow fixed bed reactor in the first reaction unit and is in a range of 0.1-2.5, preferably in a range of 0.5-2.0. For example, the second reaction unit comprises one reactor, and the reactor is provided with 1-4 catalyst bed layers.

In this preferred embodiment, the second hydrogenation reaction in step 4) is a mild liquid phase hydrogenation reaction carried out under conditions of a relatively low space velocity and a less reactor height-to-diameter ratio relative to that of the up-flow fixed bed reactor(s) in the first reaction unit, i.e. the up-flow fixed bed reactor in the second reaction unit has a suitably lower height-to-diameter ratio than that of the up-flow fixed bed reactor in the first reaction unit. The low space velocity is set mainly due to the low concentration of maleic anhydride and low reaction rate in the later stage of the maleic anhydride hydrogenation reaction, especially requiring a relatively long residence time to achieve higher conversion rate as it is difficult to convert completely when there is a small amount of residual maleic anhydride. The up-flow reaction mode not only has high mass transfer reaction efficiency, but also allows the catalyst to be in a slightly expanded state, preventing problems such as local hot spots and catalyst coking caused by long residence time. Meanwhile, due to the low concentration of residual maleic anhydride in the streams entering the up-flow fixed bed reactor in the second reaction unit, the low flow rate of the streams in the reactor under small height-to-diameter ratio conditions, and no significant turbulence of the streams, the catalyst bed layer(s) is more stable, and the streams are less prone to problems such as short-circuit flow, bubbly flow, and the like, effectively ensuring reaction efficiency and conversion capacity. The mild hydrogenation reaction in step 4) is cooperated with the rapid hydrogenation process in step 2) to ensure ideal conversion rate while effectively controlling the occurrence of side reactions, thereby improving the selectivity of the products.

According to the present application, the first and second maleic anhydride hydrogenation catalysts may each independently be a catalyst having a hydrogenation function commonly used in the hydrogenation reaction of maleic anhydride, preferably a supported nickel-based catalyst, wherein the support of the catalyst may be selected from SiO₂, Al₂O₃, SiO₂-Al₂O₃, TiO₂, activated carbon, molecular sieves or combinations thereof. The shape of the catalyst can be spherical, strip, cloverleaf or teeth-spherical, preferably spherical or teeth-spherical. Preferably, the supported nickel-based catalyst comprises 5-40% of Ni (calculated as nickel oxide) and 60-95% of the support, based on the weight of the catalyst.

In a preferred embodiment, the maleic anhydride conversion rate of the first hydrogenation reaction in step 2) is controlled to be 50-95%, preferably 55-85%.

In the process of the present application, heat removal operation may be performed between adjacent reactors as needed, and the heat removal operation may be performed by one or more of heat exchangers, air coolers, water coolers, and the like. The heat removal operation is generally controlled according to the inlet temperature of the next reactor.

In certain preferred embodiments, the process of the present application further comprises step of:
5) fractionating the second reaction effluent from the second reaction unit to obtain a succinic anhydride product.

In other preferred embodiments, the process of the present application further comprises step of:
5') subjecting the second reaction effluent from the second reaction unit to gas-liquid separation to obtain a liquid phase stream containing succinic anhydride, fractionating one part of the obtained liquid phase stream to obtain a succinic anhydride product, and recycling the remaining part of the obtained liquid phase stream back to step 2) and/or step 4) for further reaction.

In a further preferred embodiment, in step 5'), the ratio of the mass flow rate of the liquid phase stream recycled back to step 2) to the mass flow rate of the maleic anhydride solution used in step 1) is in a range of 1: 20 to 9: 10, preferably in a range of 1: 5 to 3: 5; and in step 5'), the ratio of the mass flow rate of the liquid phase stream recycled back to step 4) to the mass flow rate of the maleic anhydride solution used in step 1) is in a range of 0: 1 to 4: 5, preferably in a range of 0: 1 to 3: 10. It is further preferred that the amount of stream recycled back to step 2) is more than the amount of stream recycled back to step 4).

In a second aspect, the application provides a production system for preparing succinic anhydride by hydrogenation of maleic anhydride, comprising a first gas-liquid mixer, a first reaction unit, a second gas-liquid mixer, a second reaction unit and a fractionation device which are connected in sequence, wherein the first and second gas-liquid mixers are each provided with an inlet for liquid, an inlet for gas and an outlet for liquid phase mixture, the first and second reaction units each independently comprise one or more series-connected and/or parallel-connected up-flow fixed bed reactor(s) having arranged therein one or more fixed bed layer(s) of a maleic anhydride hydrogenation catalyst, each up-flow fixed bed reactor is provided with an inlet for hydrogenation feed and an outlet for hydrogenation product, and the fractionation device is provided with an inlet and an outlet for succinic anhydride product,
wherein the inlet for liquid of the first gas-liquid mixer is communicated with a maleic anhydride solution source, the inlet for gas is communicated with a hydrogen source, the outlet for liquid phase mixture is communicated with the inlet for hydrogenation feed of the up-flow fixed bed reactor(s) in the first reaction unit, the outlet for hydrogenation product of the up-flow fixed bed reactor(s) in the first reaction unit is communicated with the inlet for liquid of the second gas-liquid mixer, the inlet for gas of the second gas-liquid mixer is communicated with the hydrogen source, the outlet for liquid phase mixture is communicated with the inlet for hydrogenation feed of the up-flow fixed bed reactor(s) in the second reaction unit, and the outlet for hydrogenation product of the up-flow fixed bed reactor(s) in the second reaction unit is communicated with the inlet of the fractionating device,
and the height-to-diameter ratio of each up-flow fixed bed reactor in the first reaction unit is independently in a range of 3-20, preferably in a range of 4-15;
the height-to-diameter ratio of each up-flow fixed bed reactor in the second reaction unit is less than that of the up-flow fixed bed reactor in the first reaction unit, and is in a range of 0.1-2.5, preferably in a range of 0.5-2.0.

In a preferred embodiment, the production system further comprises a gas-liquid separator provided with an inlet, an outlet for gas and an outlet for liquid, wherein the outlet for hydrogenated product of the up-flow fixed bed reactor(s) in the second reaction unit is communicated with the inlet of the gas-liquid separator, the outlet for liquid of the gas-liquid separator is communicated with the inlet of the fractionation device, and the outlet for liquid of the gas-liquid separator is also communicated with the inlet for liquid of the first gas-liquid mixer and/or the second gas-liquid mixer.

In a preferred embodiment, the first and second gas-liquid mixers are each independently selected from a static mixer, an ejector mixer, a mechanical shear mixer, an impingement mixer, a microchannel mixer, or combinations thereof.

A preferred embodiment of the process and the production system for preparing succinic anhydride by hydrogenation of maleic anhydride according to the present application will be described in detail below with reference to the accompanying drawing.

As shown in FIG. 1, in a preferred embodiment, the production system for preparing succinic anhydride by hydrogenation of maleic anhydride according to the present application comprises a first gas-liquid mixer 4, a first reaction unit, a second gas-liquid mixer 11, a second reaction unit and a fractionation device (not shown in the figure) which are connected in sequence. The first gas-liquid mixer 4 and the second gas-liquid mixer 11 are each provided with an inlet for liquid, an inlet for gas and an outlet for liquid phase mixture. The first reaction unit comprises a first up-flow fixed bed reactor 6 in which a catalyst bed layer 7 is arranged; the second reaction unit comprises an up-flow fixed bed reactor 13 in which a catalyst bed layer 14 is arranged, and each of the first up-flow fixed bed reactor and the second up-flow fixed bed reactor is provided with an inlet for hydrogenation feed and an outlet for hydrogenation product. The fractionation device is provided with an inlet and an outlet for succinic anhydride product. The inlet for liquid of the first gas-liquid mixer 4 is communicated with a maleic anhydride solution source, the inlet for gas is communicated with a hydrogen source, the outlet for liquid phase mixture is communicated with the inlet for hydrogenation feed of the up-flow fixed bed reactor 6, and the outlet for hydrogenation product of the up-flow fixed bed reactor 6 is communicated with the inlet for liquid of the second gas-liquid mixer 11 through a heat remover 9. The inlet for gas of the second gas-liquid mixer is communicated with the hydrogen source, the outlet for the liquid phase mixture is communicated with the inlet for hydrogenation feed of the up-flow fixed bed reactor 13, the outlet for hydrogenation product of the up-flow fixed bed reactor 13 is communicated with the inlet of the gas-liquid separator 18 through a heat remover 16, and the outlet for liquid of the gas-liquid separator 18 is communicated with the inlet of the fractionating device.

As shown in FIG. 1, in a preferred embodiment of the process for preparing succinic anhydride by hydrogenation of maleic anhydride according to the present application, a maleic anhydride solution 1 from a maleic anhydride solution source, hydrogen 2 from a hydrogen source and a recycled stream 22 are uniformly mixed by the first gas-liquid mixer 4 to obtain a first liquid phase feed 5, which is fed into the up-flow fixed bed reactor 6 from the bottom and passed through the catalyst bed layer 7 from bottom to top to carry out a first hydrogenation reaction. A reaction effluent 8 of the up-flow fixed bed reactor 6 is fed into the heat remover 9 for heat removal. After heat removal, the reaction effluent is uniformly mixed with make-up hydrogen 10 from the hydrogen source and a recycled stream 23 by the second gas-liquid mixer 11 to obtain a second liquid phase feed 12, which is fed into the up-flow fixed bed reactor 13 from the bottom and passed through the catalyst bed layer 14 from bottom to top to carry out a second hydrogenation reaction. A reaction effluent 15 of the up-flow fixed bed reactor 13 is fed into the heat remover 16 for heat removal. The heat-removed stream 17 is fed into the gas-liquid separator 18. The gas stream 19 obtained after gas-liquid separation is withdrawn from the production system. The separated liquid stream 20 is divided into two parts, the stream 24 as one part of which is fed into the fractionating device for further separation, the stream as the other part of which is pressurized by a circulating pump 21, and then one part of the latter is introduced into the first gas-liquid mixer 4 as the recycled stream 22, and the other part of the latter is introduced into the second gas-liquid mixer 11 as the recycled stream 23.

In certain preferred embodiments, the present application provides the following embodiments:
1. A process for preparing succinic anhydride by hydrogenation of maleic anhydride, characterized by comprising following steps: feeding a first liquid phase mixed feed from bottom of an up-flow fixed bed reactor I, carrying out a rapid liquid phase hydrogenation reaction by passing through hydrogenation catalyst bed layer(s) arranged in the up-flow fixed bed reactor I from bottom to top, withdrawing the rapid hydrogenation reaction product from top of the up-flow fixed bed reactor I, after heat removal, uniformly mixing the rapid hydrogenation reaction product with make-up hydrogen to form a second liquid phase mixed feed, feeding the second liquid phase mixed feed into an up-flow fixed bed reactor II, carrying out a mild liquid phase hydrogenation reaction by passing through hydrogenation catalyst bed layer(s) arranged in the up-flow fixed bed reactor II from bottom to top, withdrawing the mild hydrogenation reaction product from top of the reactor II, after heat removal and gas phase separation, recycling one part of the mild hydrogenation reaction product to the up-flow fixed bed reactor I and/or II, and fractionating the other part of the mild hydrogenation reaction product.
2. The process according to item 1, characterized in that: the first liquid phase mixed feed and the second liquid phase mixed feed are both liquid phases with hydrogen uniformly mixed and dispersed therein, the liquid phase is a continuous phase, and the hydrogen is a dispersed phase.
3. The process according to item 1, characterized in that: the first liquid phase mixed feed and the second liquid phase mixed feed are formed by mixing with a mixer; the mixer is one or more of a static mixer, an ejector mixer, a mechanical shear mixer, an impingement mixer and a micro-channel mixer.
4. The process according to item 1, characterized in that: the fast hydrogenation reaction and the mild hydrogenation reaction are both stable whole-liquid-phase hydrogenation, with the liquid phase in the reactor being a continuous phase and the gas phase being a dispersed phase.
5. The process according to item 1, characterized in that: the maleic anhydride content in the maleic anhydride solution is 0.03-0.3 g/mL, preferably 0.05-0.20 g/mL; the solvent used in the maleic anhydride solution is one or more of benzene, toluene, xylene, acetone, tetrahydrofuran, γ-butyrolactone, methyl acetone, cyclohexanone, ethyl acetate, diethyl succinate or ethylene glycol monomethyl ether.
6. The process according to item 1, characterized in that: the hydrogen used has a purity of more than 90% by volume, preferably more than 99.9% by volume.
7. The process according to item 1, characterized in that: the rapid liquid phase hydrogenation reaction is carried out under following conditions: a reaction temperature of 40-200 °C, preferably 50-150 °C; a reaction pressure of 0.5-10.0 MPa, preferably 1-5.0 MPa; a liquid hourly space velocity of 3-20.0 h⁻¹, preferably 5.0-15.0 h⁻¹; a volume flow rate ratio of hydrogen in Nm³/h to maleic anhydride solution in m³/h in the reactor of 5: 1 to 50: 1, preferably 10: 1 to 30: 1; and a height-to-diameter ratio of the up-flow fixed bed reactor I generally being 1-15, preferably 4-10.
8. The process according to item 1, characterized in that: the up-flow fixed bed reactor I is provided with one catalyst bed layer, the catalyst is a catalyst having a hydrogenation function commonly used in the hydrogenation reaction of maleic anhydride, preferably a supported nickel-based catalyst, wherein the support of the nickel-based catalyst is one or more of SiO₂, Al₂O₃, SiO₂-Al₂O₃, TiO₂, active carbon or molecular sieves.
9. The process according to item 1, characterized in that: the mild liquid phase hydrogenation reaction is carried out under following conditions: a reaction temperature of 40-150 °C, preferably 50-80 °C; a reaction pressure of 0.5-10.0 MPa, preferably 1-5.0 MPa; a liquid hourly space velocity of 0.1-5.0 h⁻¹, preferably 0.5-2.5 h⁻¹; and a volume flow rate ratio of the hydrogen in Nm³/h in the reactor to the maleic anhydride solution in m³/h in the up-flow fixed bed reactor I of 1: 1 to 20: 1, preferably 5: 1 to 15: 1.
10. The process according to item 1, characterized in that: the up-flow fixed bed reactor II is provided with 2-4 catalyst bed layers, the catalyst is a catalyst having a hydrogenation function commonly used in the hydrogenation reaction of maleic anhydride, preferably a supported nickel-based catalyst, wherein the support of the nickel-based catalyst is one or more of SiO₂, Al₂O₃, SiO₂-Al₂O₃, TiO₂, active carbon or molecular sieves.
11. The process according to item 1, characterized in that: the rapid liquid phase hydrogenation reaction has a conversion rate of maleic anhydride of 50-95%, preferably 55-85%.
12. The process according to item 1, characterized in that: the hydrogenation reaction product recycled back to the up-flow fixed bed reactor I accounts for 5-90 wt%, preferably 20-60 wt% of the fresh stream fed into the up-flow fixed bed reactor I; the hydrogenation reaction product recycled back to the up-flow fixed bed reactor II accounts for 0-80 wt%, preferably 0-30 wt% of the fresh stream fed into the up-flow fixed bed reactor I; and the amount of stream recycled back to the up-flow fixed bed reactor I is more than the amount of stream recycled back to the up-flow fixed bed reactor II.

### Examples

The application is further illustrated by the following examples, without any intention to limit the application thereto.

The maleic anhydride raw material and the γ-butyrolactone solvent used in the following Examples and Comparative Examples were commercially available products, and their specific properties are shown in Tables 1 and 2, respectively.

**Table 1. Properties of maleic anhydride raw material**

| Item | Indicator | GB/T3676-2008 Standard Value |
|---|---|---|
| Content (in mass of maleic anhydride )/% | ≥99.5 | 99.5 |
| Color (platinum scale one), (25 °C) (140 °C, 2 hours) | ≤20 | 25 |
| | ≤40 | |
| Freezing point (lowest)/°C | 52.5 | 52.5 |
| Sulfur / ppm | ≤1 | |
| Chlorine / ppm | ≤1 | |
| Phosphorus / ppm | ≤1 | |
| Ash content / ppm | ≤10 | |
| Iron (Fe²⁺, Fe³⁺) / ppm | ≤2 | 3 |
| Stabilizing agent | Not included | |

**Table 2. Properties of γ-butyrolactone solvent**

| Item | Indicator |
|---|---|
| | Qualified product |
| γ-butyrolactone, w/% | ≥ 99.5 |
| Water, w/% | ≤ 0.1 |
| Color/Hazen unit (platinum-cobalt scale) | ≤ 30 |

The maleic anhydride hydrogenation catalysts used in the following Examples and Comparative Examples were self-made supported nickel-based catalysts, and the preparation method thereof was as follows:
A certain amount of nickel nitrate was weighed and dissolved in water to prepare a nickel nitrate solution. A certain amount of spherical alumina support was weighed, and the alumina support was impregnated in the nickel nitrate solution for a period of time, then dried at 120°C for 9 h and calcined for 6 h. The product was reduced by hydrogen at 230 °C for 72 h to obtain the catalyst.

Specific properties of the resulting catalyst are shown in Table 3.

**Table 3. Physical indicators of catalyst**

| Item | Indicator |
|---|---|
| Appearance | Spherical |
| Support | Al₂O₃ |
| Active component | Nickel |
| NiO, wt% | 28.5 |

### Example 1

According to the process of the present application, the experiment was carried out using a reaction system comprising two up-flow fixed bed reactors.

The maleic anhydride raw material was dissolved in the γ-butyrolactone solvent and mixed uniformly to obtain a maleic anhydride solution. After the solution was subjected to temperature regulation until reaching the reactor inlet temperature, it was mixed with hydrogen to obtain a liquid phase mixture. The liquid phase mixture was fed from the bottom of the first reactor and subjected to hydrogenation reaction by passing through the catalyst bed layer from bottom to top. The effluent from the first reactor was subjected to temperature regulation and then mixed with make-up hydrogen to obtain a liquid phase mixture. The liquid phase mixture was fed from the bottom of the second reactor and subjected to hydrogenation reaction by passing through the catalyst bed layer from bottom to top. The effluent from the second reactor was gas-liquid separated by a gas-liquid separator, and the separated liquid phase stream was partially recycled back to the inlet of the first and second reactors, while the remaining part was fed into a fractionation device. The specific operating conditions are shown in Table 4, and the reaction results are shown in Table 5.

### Examples 2-4

The experiment was carried out with reference to Example 1, except that the operating conditions shown in Table 4 were employed and the reaction results are shown in Table 5.

### Comparative Example 1

The experiment was carried out using two conventional down-flow trickle bed reactors.

The maleic anhydride raw material was dissolved in the γ-butyrolactone solvent and mixed uniformly to obtain a maleic anhydride solution. After the solution was subjected to temperature regulation until reaching the reactor inlet temperature and mixed with hydrogen, it was fed from the top of the first reactor and subjected to hydrogenation reaction by passing through the catalyst bed layer from top to bottom. The hydrogenation product from the first reactor was subjected to temperature regulation and then mixed with make-up hydrogen to obtain a mixture. The mixture was fed from the top of the second reactor and continuously subjected to hydrogenation reaction by passing through the catalyst bed layer from top to bottom. The effluent from the second reactor was gas-liquid separated by a gas-liquid separator, and the separated liquid phase stream was partially recycled back to the first and second reactors, while the remaining part was fed into a fractionation device. The specific operating conditions are shown in Table 4, and the reaction results are shown in Table 6.

### Comparative Example 2

The experiment was carried out using two conventional up-flow fixed bed reactors.

The maleic anhydride raw material was dissolved in the γ-butyrolactone solvent and mixed uniformly to obtain a maleic anhydride solution. After the solution was subjected to temperature regulation until reaching the reactor inlet temperature, it was mixed with hydrogen to obtain a mixture. The mixture was fed from the bottom of the first reactor and subjected to hydrogenation reaction by passing through the catalyst bed layer from bottom to top. The hydrogenation product from the first reactor was subjected to temperature regulation and then mixed with make-up hydrogen to obtain a mixture. The mixture was fed from the bottom of the second reactor and subjected to hydrogenation reaction by passing through the catalyst bed layer from bottom to top. The effluent from the second reactor was gas-liquid separated by a gas-liquid separator, and the separated liquid phase stream was partially recycled back to the first and second reactors, while the remaining part was fed into a fractionation device. The specific operating conditions are shown in Table 4, and the reaction results are shown in Table 6.

### Comparative Example 3

The experiment was carried out using a conventional up-flow fixed bed reactor in series with a conventional down-flow trickle bed reactor.

The maleic anhydride raw material was dissolved in the γ-butyrolactone solvent and mixed uniformly to obtain a maleic anhydride solution. After the solution was subjected to temperature regulation until reaching the reactor inlet temperature and mixed with hydrogen, it was fed from the bottom of the first reactor and subjected to hydrogenation reaction by passing through the catalyst bed layer from bottom to top. The hydrogenation product from the first reactor was subjected to temperature regulation and then mixed with make-up hydrogen to obtain a mixture. The mixture was fed from the top of the second reactor and subjected to hydrogenation reaction by passing through the catalyst bed layer from top to bottom. The effluent from the second reactor was gas-liquid separated by a gas-liquid separator, and the separated liquid phase stream was partially recycled back to the first and second reactors, while the remaining part was fed into a fractionation device. The specific operating conditions are shown in Table 4, and the reaction results are shown in Table 6.

### Comparative Example 4

The experiment was carried out using two conventional up-flow fixed bed reactors.

The maleic anhydride raw material was dissolved in the γ-butyrolactone solvent and mixed uniformly to obtain a maleic anhydride solution. After the solution was subjected to temperature regulation until reaching the reactor inlet temperature, it was mixed with hydrogen to obtain a mixture. The mixture was fed from the bottom of the first reactor and subjected to hydrogenation reaction by passing through the catalyst bed layer from bottom to top. The hydrogenation product from the first reactor was subjected to temperature regulation and then mixed with make-up hydrogen to obtain a mixture. The mixture was fed from the bottom of the second reactor and subjected to hydrogenation reaction by passing through the catalyst bed layer from bottom to top. The effluent from the second reactor was gas-liquid separated by a gas-liquid separator, and the separated liquid phase stream was partially recycled back to the first and second reactors, while the remaining part was fed into a fractionation device. The specific operating conditions are shown in Table 4, and the reaction results are shown in Table 6.

**Table 4. Operating conditions of Examples 1-4 and Comparative Examples 1-4**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Concentration of maleic anhydride solution | 0.15g/mL | 0.12g/mL | 0.20g/mL | 0.12g/mL | 0.12g/mL | 0.11g/mL | 0.12g/mL | 0.12g/mL |

| **First reactor** | | | | | | | | |
|---|---|---|---|---|---|---|---|---|
| Reactor type | Up-flow fixed bed reactor | | | | Down-flow trickle bed reactor | Up-flow fixed bed reactor | Up-flow fixed bed reactor | Up-flow fixed bed reactor |
| Reaction temperature, °C | 50-90 | 55-90 | 50-90 | 50-90 | 50-140 | 50-125 | 50-125 | 50-125 |
| Reaction pressure, MPaG | 4.0 | 3.5 | 3.0 | 3.0 | 6.0 | 6.0 | 6.0 | 6.5 |
| Volume space velocity, h⁻¹ | 6.0 | 8.0 | 6.0 | 150 | 2.5 | 2.5 | 2.5 | 8.0 |
| Height-to-diameter ratio of reactor | 8.0 | 8.0 | 100 | 4.0 | 2.5 | 2.5 | 2.5 | 8.0 |
| Bubble size | 50-900µm | 50-900µm | 50-900µm | 50-900µm | - | 0.5-25mm | 0.5-25mm | 0.5-25mm |
| Volume flow rate ratio of hydrogen (Nm³/h) to maleic anhydride solution (m³/h) | 22.5:1 | 22.5:1 | 35:1 | 30:1 | 300:1 | 100:1 | 100:1 | 100:1 |
| Mass flow rate ratio of recycled stream to maleic anhydride solution | 38:100 | 35:100 | 50:100 | 35:100 | 40:100 | 35:100 | 35:100 | 35:100 |

| **Second reactor** | | | | | | | | |
|---|---|---|---|---|---|---|---|---|
| Reactor type | Up-flow fixed bed reactor | | | | Down-flow trickle bed reactor | Up-flow fixed bed reactor | Down-flow trickle bed reactor | Up-flow fixed bed reactor |
| Reaction temperature, °C | 60-85 | 60-85 | 65-80 | 65-80 | 60-120 | 70-130 | 70-130 | 70-130 |
| Reaction pressure, MPaG | 3.8 | 3.3 | 2.8 | 2.8 | 5.8 | 5.8 | 5.8 | 6.3 |
| Volume space velocity, h⁻¹ | 1.5 | 1.0 | 0.8 | 0.4 | 1.0 | 1.0 | 0.8 | 1.0 |
| Height-to-diameter ratio of reactor | 2.0 | 1.5 | 1.2 | 0.8 | 2.5 | 2.5 | 2.0 | 1.0 |
| Bubble size* | 50-900µm | 50-900µm | 50-900µm | 50-900µm | - | 0.5-25mm | - | 0.5-25mm |
| Volume flow rate ratio of make-up hydrogen (Nm³/h) to maleic anhydride solution (m³/h) | 12.5:1 | 10:1 | 25:1 | 25:1 | 300:1 | 80:1 | 300:1 | 100:1 |
| Mass flow rate ratio of recycled stream to maleic anhydride solution | 20:100 | 25:100 | 50:100 | 25:100 | 30:100 | 30:100 | 25:100 | 25:100 |

| | | | | | | | | |
|---|---|---|---|---|---|---|---|---|
| *: The bubble size was measured by using an air and water system as simulation materials, introducing the gas-liquid materials according to the gas-liquid ratio used in Comparison Examples and Examples on a cold mold observation device, and using a high-speed camera to photograph the size and the distribution range of the bubbles in the reactor. | | | | | | | | |

As can be seen from Table 4, in the first and second reactors of Examples 1-4, the bubble size was only 50-900 µm, indicating that the liquid phase was the continuous phase while the gas phase was the dispersed phase, thus the reaction carried out in the reactors was whole-liquid-phase reaction. In contrast, in the first and second reactors of Comparative Examples 1-4, due to higher gas-liquid ratio (i.e., above 80:1), the gas phase was the continuous phase while the liquid phase was the dispersed phase in the reactors. That is, what was observed in the reactors was no longer bubbles, but the gas phase.

**Table 5. Reaction results of Examples 1-4**

| | Example 1 | Example 2 | Example 3 | Example 4 |
|---|---|---|---|---|
| Radial temperature difference in the first reactor, °C | 0.41 | 0.41 | 0.43 | 0.48 |
| Radial temperature difference in the second reactor, °C | 0.13 | 0.14 | 0.12 | 0.08 |
| Conversion rate in reactor I | 71.3-75.2% | 75.7-78.5% | 68.2-73.4% | 87.2-91.8% |
| Average total selectivity at average total conversion rate of about 98.0% | 99.4-99.7% | 99.3-99.7% | 99.3-99.6% | 99.5-99.9% |
| Average total selectivity at average total conversion rate of about 99.9%* | 97.0-97.4% | 97.2-97.5% | 97.2-97.6% | 97.4-98.0% |

**Table 6. Reaction results of Comparative Examples 1-4**

| | Comparative Example 1 | Comparative Example 2 | Comparative Example 3 | Comparative Example 4 |
|---|---|---|---|---|
| Radial temperature difference in the first reactor, °C | 5.6 | 6.5 | 6.2 | 5.8 |
| Radial temperature difference in the second reactor, °C | 2.2 | 2.6. | 2.7 | 2.4 |
| Conversion rate in reactor I | 84.4-87.2% | 80.0-82.5% | 80.3-84.2% | 70.6-75.0% |
| Average total selectivity at average total conversion rate of about 98.0% | 96.0-96.3% | 95.4-96.0% | 96.2-96.4% | 97.2-97.6% |
| Average total selectivity at average total conversion rate of about 99.9%* | 94.1-94.5% | 94.6-95.0% | 94.2-94.7% | 95.0-95.4% |

| | | | | |
|---|---|---|---|---|
| *In Tables 5-6, the average total conversion rate refers to the average value of the mass percentages of maleic anhydride converted relative to the maleic anhydride raw material obtained by sampling and analyzing the effluent from the second reactor every other hour during the reaction test interval; the average total selectivity refers to the average value of the percentages of the molar amount of succinic anhydride product relative to the molar amount of maleic anhydride converted by sampling and analyzing the effluent from the second reactor every other hour during the reaction test interval. | | | | |

As can be seen from the results shown in Tables 5-6, in Examples 1-4, the radial temperature differences in both the first and second reactors were less than 0.5 °C; in contrast, in Comparative Examples 1-4, the radial temperature differences in the first reactor were above 5 °C, while the radial temperature differences in the second reactor were also above 2 °C. This result shows that the process of the present application, which uses two-stage up-flow hydrogenation reactors connected in series, wherein a rapid hydrogenation reaction is carried out in the first-stage reactor under the conditions of high space velocity and large height-to-diameter ratio, while a mild hydrogenation reaction is carried out in the second-stage reactor under the conditions of low space velocity and small height-to-diameter ratio, can solve the problems of concentrated heat release and local hot spots easily occurred in the reactor due to high maleic anhydride concentration and fast reaction rate during the early stages of the reaction. Also, all of the average total selectivity at the average total conversion rate of about 98.0% and about 99.9% of Examples 1-4 was higher than that of Comparative Examples 1-4, indicating that the process of the present application can control side reactions more effectively while ensuring higher conversion rate in the later stage of the reaction, hereby high product selectivity can be obtained while achieving high conversion rate.

Meanwhile, compared with Comparative Examples 1-4, Examples 1-4 achieved better effects under the conditions of lower hydrogen-liquid ratio, indicating that the process of the present application has higher hydrogen utilization rate and can realize accurate hydrogen control. In addition, compared with Examples 1-3, Example 4 used a relatively higher hydrogen-liquid ratio within the range defined by the present application and achieved a better hydrogenation effect, further indicating that the process of the present application has high hydrogen utilization rate and more embodies the important role of the present hydrogenation process. In contrast, in Comparative Examples 1-4, although high hydrogen-liquid ratio was used, the reaction mass transfer driving force was low, the hydrogen supply amount was far greater than the chemical hydrogen consumption, resulting in low hydrogen utilization rate. The hydrogenation effect was not directly related to the hydrogen supply amount, and accurate hydrogen control and efficient hydrogenation were not achieved. In addition, as can be seen from the comparison between Example 2 and Comparative Example 4, the process of the present application can achieve better technical effects under milder reaction conditions (such as lower pressure, lower temperature and lower hydrogen-liquid ratio), resulting in lower material and energy consumption of the entire reaction system, which is also beneficial to prolonging the operating cycle of catalysts.

The present application is illustrated in detail hereinabove with reference to preferred embodiments, but is not intended to be limited to those embodiments. Various simple modifications may be made following the inventive concept of the present application, and these simple modifications shall be within the scope of the present application.

In addition, it should be noted that the various specific technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

## Claims

1. A process for preparing succinic anhydride by hydrogenation of maleic anhydride, comprising steps of:
1) mixing a maleic anhydride solution with hydrogen to obtain a first liquid phase feed with hydrogen dispersed in the liquid phase, wherein a ratio of the volume flow rate of the hydrogen in Nm³/h to the volume flow rate of the maleic anhydride solution in m³/h is in a range of 5: 1 to 50: 1, preferably 10: 1 to 30: 1;
2) carrying out a first hydrogenation reaction by passing the first liquid phase feed from bottom to top through fixed bed layer(s) of a first maleic anhydride hydrogenation catalyst arranged in a first reaction unit under first hydrogenation reaction conditions to obtain a first reaction effluent containing succinic anhydride, wherein the first hydrogenation reaction conditions include: a reaction temperature of 40-200 °C, preferably 50-150 °C; a reaction pressure of 0.5-10.0 MPa, preferably 1-5.0 MPa; and a liquid hourly space velocity of 5.0-20.0 h⁻¹, preferably 6.0-15.0 h⁻¹;
3) mixing the first reaction effluent from the first reaction unit with make-up hydrogen to obtain a second liquid phase feed with hydrogen dispersed in the liquid phase, wherein a ratio of the volume flow rate of the make-up hydrogen in Nm³/h to the volume flow rate of the maleic anhydride solution used in step 1) in m³/h is in a range of 1: 1 to 20: 1, preferably 5: 1 to 15: 1; and
4) carrying out a second hydrogenation reaction by passing the second liquid phase feed from bottom to top through fixed bed layer(s) of a second maleic anhydride hydrogenation catalyst arranged in a second reaction unit under second hydrogenation reaction conditions to obtain a second reaction effluent containing succinic anhydride, wherein the second hydrogenation reaction conditions include: a reaction temperature of 40-150 °C, preferably 50-80 °C; a reaction pressure of 0.5-10.0 MPa, preferably 1-5.0 MPa; and a liquid hourly space velocity of 0.1-4.0 h⁻¹, preferably 0.5-2.5 h⁻¹.

2. The process according to claim 1, wherein the first reaction unit comprises one or more up-flow fixed bed reactor(s) having arranged therein one or more fixed bed layer(s) of the first maleic anhydride hydrogenation catalyst, wherein a height-to-diameter ratio of each up-flow fixed bed reactor is independently in a range of 3-20, preferably in a range of 4-15.

3. The process according to claim 2, wherein the second reaction unit comprises one or more up-flow fixed bed reactor(s) having arranged therein one or more fixed bed layer(s) of the second maleic anhydride hydrogenation catalyst, wherein a height-to-diameter ratio of each up-flow fixed bed reactor in the second reaction unit is less than that of the up-flow fixed bed reactor in the first reaction unit and is in a range of 0.1-2.5, preferably in a range of 0.5-2.0.

4. The process according to any one of the preceding claims, wherein the first and second maleic anhydride hydrogenation catalysts are each independently a supported nickel-based catalyst, wherein a support of the nickel-based catalyst is selected from SiO₂, Al₂O₃, SiO₂-Al₂O₃, TiO₂, activated carbon, molecular sieves, or combinations thereof; preferably, the supported nickel-based catalyst comprises 5-40% of Ni (calculated as nickel oxide) and 60-95% of the support, based on the weight of the catalyst.

5. The process according to any one of the preceding claims, wherein the maleic anhydride solution used in step 1) has a maleic anhydride content of 0.03-0.3 g/mL, preferably 0.05-0.2 g/mL;
preferably, a solvent used in the maleic anhydride solution is selected from benzene, toluene, xylene, acetone, tetrahydrofuran, γ-butyrolactone, methyl acetone, cyclohexanone, ethyl acetate, diethyl succinate, ethylene glycol monomethyl ether, or combinations thereof.

6. The process according to any one of the preceding claims, wherein a maleic anhydride conversion rate of the first hydrogenation reaction in step 2) is controlled to be 50-95%, preferably 55-85%.

7. The process according to any one of the preceding claims, further comprising step of:
5) fractionating the second reaction effluent from the second reaction unit to obtain a succinic anhydride product; or alternatively
5') subjecting the second reaction effluent from the second reaction unit to gas-liquid separation to obtain a liquid phase stream containing succinic anhydride, fractionating one part of the obtained liquid phase stream to obtain a succinic anhydride product, and recycling the remaining part of the obtained liquid phase stream back to step 2) and/or step 4) for further reaction.

8. The process according to claim 7, wherein:
in step 5'), a ratio of the mass flow rate of the liquid phase stream recycled back to step 2) to the mass flow rate of the maleic anhydride solution used in step 1) is in a range of 1: 20 to 9: 10, preferably in a range of 1: 5 to 3: 5; and
in step 5'), a ratio of the mass flow rate of the liquid phase stream recycled back to step 4) to the mass flow rate of the maleic anhydride solution used in step 1) is in a range of 0: 1 to 4: 5, preferably in a range of 0: 1 to 3: 10;
preferably, the mass flow rate of the liquid phase stream recycled back to step 2) is greater than the mass flow rate of the liquid phase stream recycled back to step 4).

9. A production system for preparing succinic anhydride by hydrogenation of maleic anhydride, comprising a first gas-liquid mixer, a first reaction unit, a second gas-liquid mixer, a second reaction unit and a fractionating device which are connected in sequence, wherein the first and second gas-liquid mixers are each provided with an inlet for liquid, an inlet for gas and an outlet for liquid phase mixture, the first and second reaction units each independently comprise one or more series-connected and/or parallel-connected up-flow fixed bed reactors having arranged therein one or more fixed bed layer(s) of a maleic anhydride hydrogenation catalyst, each up-flow fixed bed reactor is provided with an inlet for hydrogenation feed and an outlet for hydrogenation product, and the fractionating device is provided with an inlet and an outlet for succinic anhydride product,
wherein the inlet for liquid of the first gas-liquid mixer is communicated with a maleic anhydride solution source, the inlet for gas is communicated with a hydrogen source, the outlet for liquid phase mixture is communicated with the inlet for hydrogenation feed of the up-flow fixed bed reactor(s) in the first reaction unit, the outlet for hydrogenation product of the up-flow fixed bed reactor(s) in the first reaction unit is communicated with the inlet for liquid of the second gas-liquid mixer, the inlet for gas of the second gas-liquid mixer is communicated with the hydrogen source, the outlet for liquid phase mixture is communicated with the inlet for hydrogenation feed of the up-flow fixed bed reactor(s) in the second reaction unit, and the outlet for hydrogenation product of the up-flow fixed bed reactor(s) in the second reaction unit is communicated with the inlet of the fractionating device,
and a height-to-diameter ratio of each up-flow fixed bed reactor in the first reaction unit is independently in a range of 3-20, preferably in a range of 4-15;
and a height-to-diameter ratio of each up-flow fixed bed reactor in the second reaction unit is less than that of the up-flow fixed bed reactor in the first reaction unit, and is in a range of 0.1-2.5, preferably in a range of 0.5-2.0.

10. The production system according to claim 9, further comprising a gas-liquid separator provided with an inlet, an outlet for gas, and an outlet for liquid, wherein the outlet for hydrogenated product of the up-flow fixed bed reactor(s) in the second reaction unit is communicated with the inlet of the gas-liquid separator, the outlet for liquid of the gas-liquid separator is communicated with the inlet of the fractionation device, and the outlet for liquid of the gas-liquid separator is also communicated with the inlet for liquid of the first gas-liquid mixer and/or the second gas-liquid mixer.

11. The production system according to claim 9 or 10, wherein the first and second gas-liquid mixers are each independently selected from a static mixer, an ejector mixer, a mechanical shear mixer, an impingement mixer, a microchannel mixer, or combinations thereof.
